(19) Europäisches Patentamt
European Patent Office
Office européen des brevets

(11) EP 0 882 792 A2

(12) EUROPEAN PATENT APPLICATION

(43) Date of publication:
09.12.1998 Bulletin 1998/50

(51) Int Cl.6: C12N 15/12, C07K 14/47, C12N 5/10, C07K 16/18

(21) Application number: 98304207.8

(22) Date of filing: 28.05.1998

(84) Designated Contracting States:
AT BE CH CY DE DK ES FI FR GB GR IE IT LI LU MC NL PT SE
Designated Extension States:
AL LT LV MK RO SI

(30) Priority: 06.06.1997 EP 97401277
26.12.1997 EP 97403166
26.12.1997 EP 97403165

(71) Applicants:
- SMITHKLINE BEECHAM LABORATOIRES PHARMACEUTIQUES 92731 Nanterre Cédex (FR)
- SMITHKLINE BEECHAM CORPORATION Philadelphia Pennsylvania 19101 (US)

(72) Inventors:
- Bril, Antoine M.A., SmithKline Beecham Lab. Pharm. 35762 Saint Gregoire (FR)
- Calmels, T.G.G., SmithKline Beecham Lab. Pharm. 35762 Saint Gregoire (FR)
- Hurle, Mark R., SmithKline Beecham Pharm. King of Prussia, PA 19406 (US)
- Leger, Isabelle M., SmithKline Beecham Lab. Pharm. 35762 Saint Gregoire (FR)
- Souchet, Michel L., SmithKline Beecham Lab. Pharm. 35762 Saint Gregoire (FR)
- Tourtelier, L.N.P., SmithKline Beecham Lab. Pharm. 35762 Saint Gregoire (FR)

(74) Representative: Crump, Julian Richard John et al
FJ Cleveland,
40-43 Chancery Lane
London WC2A 1JQ (GB)

# (54) CSB5, a member of the guanine nucleotide exchange factor (GEF) family of polypeptides

(57) CSB5 polypeptides and polynucleotides and methods for producing such polypeptides by recombinant techniques are disclosed. Also disclosed are methods for utilizing CSB5 polypeptides and polynucleotides in therapy, and diagnostic assays for such.



Printed by Jouve, 75001 PARIS (FR)

## Description

### Field of the Invention

This invention relates to newly identified polypeptides and polynucleotides encoding such polypeptides, to their use in therapy and in identifying compounds which may be agonists, antagonists and/or inhibitors which are potentially useful in therapy, and to production of such polypeptides and polynucleotides.

### Background of the Invention

The drug discovery process is currently undergoing a fundamental revolution as it embraces 'functional genomics', that is, high throughput genome- or gene-based biology. This approach as a means to identify genes and gene products as therapeutc targets is rapidly superceding earlier approaches based on 'positional cloning'. A phenotype, that is a biological function or genetic disease, would be identified and this would then be tracked back to the responsible gene, based on its genetic map position. Functional genomics relies heavily on high-throughput DNA sequencing technologies and the various tools of bioinformatics to identify gene sequences of potential interest from the many molecular biology databases now available. There is a continuing need to identify and characterise further genes and their related polypeptides/proteins, as targets for drug discovery.

### Summary of the Invention

The present invention relates to CSB5, in particular CSB5 polypeptides and CSB5 polynucleotides, recombinant materials and methods for their production. In another aspect, the invention relates to methods for using such polypeptides and polynucleotides, including the treatment of heart hypertrophy, heart failure, ischemia, arrythmia, hypertension, atherosclerosis, restenosis, chronic and acute inflammation, cerebral stroke, rheumatoid arthritis, multiple sclerosis, bowel diseases, diabetes, cancer, among others , hereinafter referred to as "the diseases", amongst others. In a further aspect, the invention relates to methods for identifying agonists and antagonists/inhibitors using the materials provided by the invention, and treating conditions associated with CSB5 imbalance with the identified compounds. In a still further aspect, the invention relates to diagnostic assays for detecting diseases associated with inappropriate CSB5 activity or levels.

### Description of the Invention

In a first aspect, the present invention relates to CSB5 polypeptides. Such peptides include isolated polypeptides comprising an amino acid sequence which has at least 70% identity, preferably at least 80% identity, more preferably at least 90% identity, yet more preferably at least 95% identity, most preferably at least 97-99% identity, to that of SEQ ID NO:2 or SEQ ID NO:6 over the entire length of SEQ ID NO:2 or SEQ ID NO:6. Such polypeptides include those comprising the amino acid sequences of SEQ ID NO:2 or SEQ ID NO:6. A comparison of the polypeptides of SEQ ID NO:2 (CSB5) and SEQ ID NO:6 (CSB5var) shows that SEQ ID NO:2 has a 30 amino acid insertion relative to SEQ ID NO:6, between amino acid residues 243 and 244 of SEQ ID NO:6, which may indicate that these two polypeptides are splice variants or that the polypeptide of SEQ ID NO:2 results from translation of a mRNA transcript that comprises at least one unspliced intron.

Further peptides of the present invention include isolated polypeptides in which the amino acid sequence has at least 70% identity, preferably at least 80% identity, more preferably at least 90% identity, yet more preferably at least 95% identity, most preferably at least 97-99% identity, to the amino acid sequences of SEQ ID NO:2 or SEQ ID NO:6 over the entire length of SEQ ID NO:2 or SEQ ID NO:6. Such polypeptides include the polypeptides of SEQ ID NO:2 and SEQ ID NO:6.

Further peptides of the present invention include isolated polypeptides encoded by a polynucleotide comprising the sequence contained in SEQ ID NO:1 or SEQ ID NO:5.

Polypeptides of the present invention are believed to be members of the guanine nucleotide exchange factor (GEF) family of polypeptides. They are therefore of interest because GEF proteins regulate the RAS superfamily of GTPases which are involved in cytoskeletal organisation, cell growth and proliferation, cell migration, cell adhesion, membrane trafficking, vesicle transport, mitosis, ADP-ribosylation, phospholipid metabolism and programmed cell death, among other things. These properties are hereinafter referred to as "CSB5 activity" or "CSB5 polypeptide activity" or "biological activity of CSB5". Also included amongst these activities are antigenic and immunogenic activities of said CSB5 polypeptides, in particular the antigenic and immunogenic activities of the polypeptides of SEQ ID NO:2 or SEQ ID NO:6. Preferably, a polypeptide of the present invention exhibits at least one biological activity of CSB5.

The polypeptides of the present invention may be in the form of the "mature" protein or may be a part of a larger protein such as a precursor or a fusion protein. It is often advantageous to include an additional amino acid sequence which contains secretory or leader sequences, prosequences, sequences which aid in purification such as multiple histidine residues, or an additional sequence for stability during recombinant production.

The present invention also includes variants of the aforementioned polypetides, that is polypeptides that vary from the referents by conservative amino acid substitutions, whereby a residue is substituted by another with like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, 1-3, 1-2 or 1 amino acids are substituted, deleted, or added in any combination.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

In a further aspect, the present invention relates to CSB5 polynucleotides. Such polynucleotides include isolated polynucleotides comprising a nucleotide sequence encoding a polypeptide which has at least 70% identity, preferably at least 80% identity, more preferably at least 90% identity, yet more preferably at least 95% identity, to the amino acid sequence of SEQ ID NO:2 or SEQ ID NO:6, over the entire length of SEQ ID NO:2 or SEQ ID NO:6. In this regard, polypeptides which have at least 97% identity are highly preferred, whilst those with at least 98-99% identity are more highly preferred, and those with at least 99% identity are most highly preferred. Such polynucleotides include a polynucleotide comprising the nucleotide sequence contained in SEQ ID NO: 1 encoding the polypeptide of SEQ ID NO: 2 and a polynucleotide comprising the nucleotide sequence contained in SEQ ID NO:5 encoding the polypeptide of SEQ ID NO:6.

Further polynucleotides of the present invention include isolated polynucleotides comprising a nucleotide sequence that has at least 70% identity, preferably at least 80% identity, more preferably at least 90% identity, yet more preferably at least 95% identity, to a nucleotide sequence encoding a polypeptide of SEQ ID NO:2 or SEQ ID NO:6, over the entire coding region. In this regard, polynucleotides which have at least 97% identity are highly preferred, whilst those with at least 98-99% identity are more highly preferred, and those with at least 99% identity are most highly preferred.

Further polynucleotides of the present invention include isolated polynucleotides comprising a nucleotide sequence which has at least 70% identity, preferably at least 80% identity, more preferably at least 90% identity, yet more preferably at least 95% identity, to SEQ ID NO:1 or SEQ ID NO:5 over the entire length of SEQ ID NO:1 or SEQ ID NO:5. In this regard, polynucleotides which have at least 97% identity are highly preferred, whilst those with at least 98-99% identity are more highly preferred, and those with at least 99% identity are most highly preferred. Such polynucleotides include a polynucleotide comprising the polynucleotide of SEQ ID NO:1 or SEQ ID NO:5 as well as the polynucleotides of SEQ ID NO: 1 and SEQ ID NO:5.

The invention also provides polynucleotides which are complementary to all the above described polynucleotides.

The nucleotide sequences of SEQ ID NO:1 and SEQ ID NO:5 show homology with human Trio (Debant, A. *et. al.,* Proc.Nat.Acad.Sci. USA 99, 5466-5471, 1996; GenBank Accession No. U42390). The nucleotide sequence of SEQ ID NO:1 is a cDNA sequence and comprises a polypeptide encoding sequence (nucleotide 88 to 1830) encoding a polypeptide of 580 amino acids, the polypeptide of SEQ ID NO:2. The nucleotide sequence of SEQ ID NO:5 is a cDNA sequence and comprises a polypeptide encoding sequence (nucleotide 88 to 1740) encoding a polypeptide of 550 amino acids, the polypeptide of SEQ ID NO:6. The nucleotide sequence encoding the polypeptide of SEQ ID NO:2 may be identical to the polypeptide encoding sequence contained in SEQ ID NO:1 or it may be a sequence other than the one contained in SEQ ID NO:1, which, as a result of the redundancy (degeneracy) of the genetic code, also encodes the polypeptide of SEQ ID NO:2. Similarly the nucleotide sequence encoding the polypeptide of SEQ ID NO:6 may be identical to the polypeptide encoding sequence contained in SEQ ID NO:5 or it may be a sequence other than the one contained in SEQ ID NO:5, which, as a result of the redundancy (degeneracy) of the genetic code, also encodes the polypeptide of SEQ ID NO:6.

The polypeptides of SEQ ID NO:2 and SEQ ID NO:6 are structurally related to other proteins of the guanine nucleotide exchange factor (GEF) family, having homology and/or structural similarity with human Trio (Debant, A. *et. al.,* Proc.Nat.Acad.Sci. USA 99, 5466-5471, 1996; GenBank Accession No. U42390).

Preferred polypeptides and polynucleotides of the present invention are expected to have. *inter alia,* similar biological functions/properties to their homologous polypeptides and polynucleotides. Furthermore, preferred polypeptides and polynucleotides of the present invention have at least one CSB5 activity.

The present invention also relates to partial or other polynucleotide and polypeptide sequences which were first identified prior to the determination of the corresponding full length sequences of SEQ ID NO:1, SEQ ID NO:5, SEQ ID NO:2 and SEQ ID NO:6.

Accordingly, in a further aspect, the present invention provides for an isolated polynucleotide which:

(a) comprises a nucleotide sequence which has at least 75% identity, preferably at least 80% identity, more preferably at least 90% identity, yet more preferably at least 95% identity, even more preferably at least 97-99% identity to SEQ ID NO:3 over the entire length of SEQ ID NO:3;
(b) has a nucleotide sequence which has at least 75% identity, preferably at least 80% identity, more preferably at least 90% identity, yet more preferably at least 95% identity, even more preferably at least 97-99% identity, to SEQ ID NO:3 over the entire length of SEQ ID NO:3;
(c) the polynucleotide of SEQ ID NO:3; or
(d) a nucleotide sequence encoding a polypeptide which has at least 70% identity, preferably at least 80% identity, more preferably at least 90% identity, yet more preferably at least 95% identity, even more preferably at least 97-99% identity, to the amino acid sequence of SEQ ID NO:4, over the entire length of SEQ ID NO:4;

as well as the polynucleotide of SEQ ID NO:3.

The present invention further provides for a polypeptide which:

(a) comprises an amino acid sequence which has at least 70% identity, preferably at least 80% identity, more preferably at least 90% identity, yet more preferably at least 95% identity, most preferably at least 97-99% identity, to that of SEQ ID NO:4 over the entire length of SEQ ID NO:4;
(b) has an amino acid sequence which has at least 70% identity, preferably at least 80% identity, more preferably at least 90% identity, yet more preferably at least 95% identity, most preferably at least 97-99% identity, to the amino acid sequence of SEQ ID NO:4 over the entire length of SEQ ID NO:4;
(c) comprises the amino acid of SEQ ID NO:4; and
(d) is the polypeptide of SEQ ID NO:4;

as well as polypeptides encoded by a polynucleotide comprising the sequence contained in SEQ ID NO:3.

The nucleotide sequence of SEQ ID NO:3 and the peptide sequences encoded thereby are derived from EST (Expressed Sequence Tag) sequences. It is recognised by those skilled in the art that there will inevitably be some nucleotide sequence reading errors in EST sequences (see Adams, M.D. *et al,* Nature 377 (supp) 3, 1995). Accordingly, the nucleotide sequence of SEQ ID NO:3 and the peptide sequences encoded therefrom are therefore subject to the same inherent limitations in sequence accuracy. Furthermore, the peptide sequences encoded by SEQ ID NO:3 comprise a region of identity or close homology and/or close structural similarity (for example a conservative amino acid difference) with the closest homologous or structurally similar protein.

Polynucleotides of the present invention may be obtained, using standard cloning and screening techniques, from a cDNA library derived from mRNA in cells of human brain, heart, foetal dura mater, testis, Hodgkinson's lymphoma II, pancreas islet cell tumour or uterus, using the expressed sequence tag (EST) analysis (Adams, M.D., *et al.* Science (1991) 252:1651-1656; Adams, M.D. *et al.,* Nature, (1992) *355*:632-634; Adams, M.D., *et al.,* Nature (1995) 377 Supp: 3-174). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself; or the coding sequence for the mature polypeptide in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al.,* Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further embodiments of the present invention include polynucleotides encoding polypeptide variants which comprise the amino acid sequences of SEQ ID NO:2 and SEQ ID NO:6 and in which several, for instance from 5 to 10, 1 to 5, 1 to 3, 1 to 2 or 1, amino acid residues are substituted, deleted or added, in any combination.

Polynucleotides which are identical or sufficiently identical to a nucleotide sequence contained in SEQ ID NO:1 or SEQ ID NO:5, may be used as hybridization probes for cDNA and genomic DNA or as primers for a nucleic acid amplification (eg. PCR) reaction, to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1 or SEQ ID NO:5. Typically these nucleotide sequences are 70% identical, preferably 80% identical, more preferably 90% identical, most preferably 95% identical to that of the referent. The probes or primers will generally comprise at least 15 nucleotides, preferably, at least 30 nucleotides and may have at least 50 nucleotides. Particularly preferred probes will have between 30 and 50 nucleotides. Particularly preferred primers will have between 20 and 25 nucleotides.

A polynucleotide encoding a polypeptide of the present invention, including homologs from species other than human, may be obtained by a process which comprises the steps of screening an appropriate library under stringent hybridization conditions with a labeled probe having the sequence of SEQ ID NO:1, SEQ ID NO:5 or fragments thereof; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA; followed by washing the filters in 0.1x SSC at about 65°C. Thus the present invention also includes polynucleotides obtainable by screening an appropriate library under stringent hybridization conditions with a labeled probe having the sequence of SEQ ID NO:1, SEQ ID NO:5 or fragments thereof.

The skilled artisan will appreciate that, in many cases, an isolated cDNA sequence will be incomplete, in that the region coding for the polypeptide is short at the 5' end of the cDNA. This is a consequence of reverse transcriptase, an enzyme with inherently low 'processivity' (a measure of the ability of the enzyme to remain attached to the template during the polymerisation reaction), failing to complete a DNA copy of the mRNA template during 1 st strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., PNAS USA 85, 8998-9002, 1988). Recent modifications of the technique, exemplified by the Marathon™ technology (Clontech Laboratories Inc.) for example, have significantly simplified the search for longer cDNAs. In the Marathon™ technology, cDNAs have been prepared from mRNA extracted from a chosen tissue and an 'adaptor' sequence ligated onto each end. Nucleic acid amplification (PCR) is then carried out to amplify the 'missing' 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, in a further aspect, the present invention relates to expression systems which comprise a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., Basic Methods in Molecular Biology (1986) and Sambrook *et al.,* Molecular Cloning: A Laboratory Manual, 2nd Ed., Cold Spring Harbor Laboratory Press, Cold Spring Harbor, N.Y. (1989). Preferred such methods include, for instance, calcium phosphate transfection, DEAE-dextran mediated transfection, transfection, microinjection, cationic lipid-mediated transfection, electroporation, transduction, scrape loading, ballistic introduction or infection.

Representative examples of appropriate hosts include bacterial cells, such as *Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector which is able to maintain, propagate or express a polynucleotide to produce a polypeptide in a host may be used. The appropriate nucleotide sequence may be inserted into an expression system by any of a variety of well-known and routine techniques, such as, for example, those set forth in Sambrook *et al.,* Molecular Cloning, A Laboratory Manual (supra). Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and or purification.

This invention also relates to the use of polynucleotides of the present invention as diagnostic reagents. Detection of a mutated form of the gene characterised by the polynucleotides of SEQ ID NO: 1 or SEQ ID NO:5 which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled CSB5 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing (ee, e.g., Myers *et al.,* Science (1985) 230:1242). Sequence changes at specific locations may also be revealed by nuclease protection assays, such as RNase and S1 protection or the chemical cleavage method (see Cotton *et al.,* Proc Natl Acad Sci USA (1985) 85: 4397-4401). In another embodiment, an array of oligonucleotides probes comprising CSB5 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability (see for example: M.Chee et al., Science, Vol 274, pp 610-613 (1996)).

The diagnostic assays offer a process for diagnosing or determining a susceptibility to the diseases through detection of mutation in the CSB5 gene by the methods described. In addition, such diseases may be diagnosed by methods comprising determining from a sample derived from a subject an abnormally decreased or increased level of polypeptide or mRNA. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention relates to a diagonostic kit which comprises:

(a) a polynucleotide of the present invention, preferably the nucleotide sequence of SEQ ID NO: 1, SEQ ID NO:5 or a fragment thereof;
(b) a nucleotide sequence complementary to that of (a);
(c) a polypeptide of the present invention, preferably the polypeptide of SEQ ID NO:2, SEQ ID NO:6 or a fragment thereof; or
(d) an antibody to a polypeptide of the present invention, preferably to the polypeptide of SEQ ID NO:2 or SEQ ID NO:6.

It will be appreciated that in any such kit, (a), (b), (c) or (d) may comprise a substantial component. Such a kit will be of use in diagnosing a disease or suspectability to a disease, particularly heart hypertrophy, heart failure, ischemia, arrythmia, hypertension, atherosclerosis, restenosis, chronic and acute inflammation, cerebral stroke, theumatoid arthritis, multiple sclerosis, bowel diseases, diabetes, cancer, amongst others.

The nucleotide sequences of the present invention are also valuable for chromosome localisation . The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The nucleotide sequences of the present invention are also valuable for tissue localisation. Such techniques allow the determination of expression patterns of the CSB5 polypeptides in tissues by detection of the mRNAs that encode them. These techniques include in situ hybridziation techniques and nucleotide amplification techniques, for example PCR. Such techniques are well known in the art. Results from these studies provide an indication of the normal functions of the polypeptides in the organism. In addition, comparative studies of the normal expression pattern of CSB5 mRNAs with that of mRNAs encoded by a mutant CSB5 gene provide valuable insights into the role of mutant CSB5 polypeptides, or that of inappropriate expression of normal CSB5 polypeptides, in disease. Such inappropriate expression may be of a temporal, spatial or simply quantitative nature.

The polypeptides of the invention or their fragments or analogs thereof, or cells expressing them, can also be used as immunogens to produce antibodies immunospecific for polypeptides of the present invention. The term "immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, analogs or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozbor *et al.,* Immunology Today (1983) 4:72) and the EBV-hybridoma technique (Cole *et al.,* Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography.

Antibodies against polypeptides of the present invention may also be employed to treat the diseases, amongst others.

In a further aspect, the present invention relates to genetically engineered soluble fusion proteins comprising a polypeptide of the present invention, or a fragment thereof, and various portions of the constant regions of heavy or light chains of immunoglobulins of various subclasses (IgG, IgM, IgA, IgE). Preferred as an immunoglobulin is the constant part of the heavy chain of human IgG, particularly IgG1, where fusion takes place at the hinge region. In a particular embodiment, the Fc part can be removed simply by incorporation of a cleavage sequence which can be cleaved with blood clotting factor Xa. Furthermore, this invention relates to processes for the preparation of these fusion proteins by genetic engineering, and to the use thereof for drug screening, diagnosis and therapy. A further aspect of the invention also relates to polynucleotides encoding such fusion proteins. Examples of fusion protein technology can be found in International Patent Application Nos. WO94/29458 and WO94/22914.

Another aspect of the invention relates to a method for inducing an immunological response in a mammal which comprises inoculating the mammal with a polypeptide of the present invention, adequate to produce antibody and/or T cell immune response to protect said animal from the diseases hereinbefore mentioned, amongst others. Yet another aspect of the invention relates to a method of inducing immunological response in a mammal which comprises, delivering a polypeptide of the present invention *via* a vector directing expression of the polynucleotide and coding for the polypeptide *in vivo* in order to induce such an immunological response to produce antibody to protect said animal from diseases.

A further aspect of the invention relates to an immunological/vaccine formulation (composition) which, when introduced into a mammalian host, induces an immunological response in that mammal to a polypeptide of the present invention wherein the composition comprises a polypeptide or polynucleotide of the present invention. The vaccine formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention are responsible for one or more biological functions, including one or more disease states, in particular the diseases hereinbefore mentioned. It is therefore desirous to devise screening methods to identify compounds which stimulate or which inhibit the function of the polypeptide. Accordingly, in a further aspect, the present invention provides for a method of screening compounds to identify those which stimulate or which inhibit the function of the polypeptide. In general, agonists or antagonists may be employed for therapeutic and prophylactic purposes for such diseases as hereinbefore mentioned. Compounds may be identified from a variety of sources, for example, cells, cell-free preparations, chemical libraries, and natural product mixtures. Such agonists, antagonists or inhibitors so-identified may be natural or modified substrates, ligands, receptors, enzymes, etc., as the case may be, of the polypeptide; or may be structural or functional mimetics thereof (see Coligan *et al.,* Current Protocols in Immunology 1(2):Chapter 5 (1991)).

The screening method may simply measure the binding of a candidate compound to the polypeptide, or to cells or membranes bearing the polypeptide, or a fusion protein thereof by means of a label directly or indirectly associated with the candidate compound. Alternatively, the screening method may involve competition with a labeled competitor. Further, these screening methods may test whether the candidate compound results in a signal generated by activation or inhibition of the polypeptide. using detection systems appropriate to the cells bearing the polypeptide. Inhibitors of activation are generally assayed in the presence of a known agonist and the effect on activation by the agonist by the presence of the candidate compound is observed. Constitutively active polypeptides may be employed in screening methods for inverse agonists or inhibitors, in the absence of an agonist or inhibitor, by testing whether the candidate compound results in inhibition of activation of the polypeptide. Further, the screening methods may simply comprise the steps of mixing a candidate compound with a solution containing a polypeptide of the present invention, to form a mixture, measuring CSB5 activity in the mixture, and comparing the CSB5 activity of the mixture to a standard. Fusion proteins, such as those made from Fc portion and CSB5 polypeptide, as hereinbefore described, can also be used for high-throughput screening assays to identify antagonists for the polypeptide of the present invention (see D. Bennett *et al.,* J Mol Recognition, 8:52-58 (1995); and K. Johanson *et al.,* J Biol Chem, 270(16):9459-9471 (1995)).

The polynucleotides, polypeptides and antibodies to the polypeptide of the present invention may also be used to configure screening methods for detecting the effect of added compounds on the production of mRNA and polypeptide in cells. For example, an ELISA assay may be constructed for measuring secreted or cell associated levels of polypeptide using monoclonal and polyclonal antibodies by standard methods known in the art. This can be used to discover agents which may inhibit or enhance the production of polypeptide (also called antagonist or agonist, respectively) from suitably manipulated cells or tissues.

The polypeptide may be used to identify membrane bound or soluble receptors, if any, through standard receptor binding techniques known in the art. These include, but are not limited to, ligand binding and crosslinking assays in which the polypeptide is labeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide which compete with the binding of the polypeptide to its receptors, if any. Standard methods for conducting such assays are well understood in the art.

Examples of potential polypeptide antagonists include antibodies or, in some cases, oligonucleotides or proteins which are closely related to the ligands, substrates, receptors, enzymes, etc., as the case may be, of the polypeptide, e.g., a fragment of the ligands, substrates, receptors, enzymes, etc.; or small molecules which bind to the polypeptide of the present invention but do not elicit a response, so that the activity of the polypeptide is prevented.

Thus, in another aspect, the present invention relates to a screening kit for identifying agonists, antagonists, ligands, receptors, substrates, enzymes, etc. for polypeptides of the present invention; or compounds which decrease or enhance the production of such polypeptides, which comprises:

(a) a polypeptide of the present invention;
(b) a recombinant cell expressing a polypeptide of the present invention;
(c) a cell membrane expressing a polypeptide of the present invention; or
(d) antibody to a polypeptide of the present invention;

which polypeptide is preferably that of SEQ ID NO:2 or SEQ ID NO:6.

It will be appreciated that in any such kit, (a), (b), (c) or (d) may comprise a substantial component.

It will be readily appreciated by the skilled artisan that a polypeptide of the present invention may also be used in a method for the structure-based design of an agonist, antagonist or inhibitor of the polypeptide, by:

(a) determining in the first instance the three-dimensional structure of the polypeptide;

(b) deducing the three-dimensional structure for the likely reactive or binding site(s) of an agonist, antagonist or inhibitor;

(c) synthesing candidate compounds that are predicted to bind to or react with the deduced binding or reactive site; and

(d) testing whether the candidate compounds are indeed agonists, antagonists or inhibitors. It will be further appreciated that this will normally be an iterative process.

In a further aspect, the present invention provides methods of treating abnormal conditions such as, for instance, heart hypertrophy, heart failure, ischemia, arrythmia, hypertension, atherosclerosis, restenosis, chronic and acute inflammation, cerebral stroke, theumatoid arthritis, multiple sclerosis, bowel diseases, diabetes, cancer, related to either an excess of, or an under-expression of, CSB5 polypeptide activity.

If the activity of the polypeptide is in excess, several approaches are available. One approach comprises administering to a subject in need thereof an inhibitor compound (antagonist) as hereinabove described, optionally in combination with a pharmaceutically acceptable carrier, in an amount effective to inhibit the function of the polypeptide, such as, for example, by blocking the binding of ligands, substrates, receptors, enzymes, etc., or by inhibiting a second signal, and thereby alleviating the abnormal condition. In another approach, soluble forms of the polypeptides still capable of binding the ligand, substrate, enzymes, receptors, etc. in competition with endogenous polypeptide may be administered. Typical examples of such competitors include fragments of the CSB5 polypeptide.

In still another approach, expression of the gene encoding endogenous CSB5 polypeptide can be inhibited using expression blocking techniques. Known such techniques involve the use of antisense sequences, either internally generated or externally administered (see, for example, O'Connor, J Neurochem (1991) 56:560 in Oligodeoxynucleotides as Antisense Inhibitors of Gene Expression, CRC Press, Boca Raton, FL (1988)). Alternatively, oligonucleotides which form triple helices ("triplexes") with the gene can be supplied (see, for example, Lee *et al.,* Nucleic Acids Res (1979) 6:3073; Cooney *et al.,* Science (1988) 241:456; Dervan *et al.,* Science (1991) 251:1360). These oligomers can be administered *per se* or the relevant oligomers can be expressed *in vivo.* Synthetic antisense or triplex oligonucleotides may comprise modified bases or modified backbones. Examples of the latter include methylphosphonate, phosphorothioate or peptide nucleic acid (PNA) backbones. Such backbones are incorporated in the antisense or triplex oligonucleotide in order to provide protection from degradation by nucleases and are well known in the art. Antisense and triplex molecules synthesised with these or other modified backbones also form part of the present invention.

In addition, expression of the CSB5 polypeptide may be prevented by using ribozymes specific to the CSB5 mRNA sequence. Ribozymes are catalytically active RNAs that can be natural or synthetic (see for example Usman, N, et al., Curr. Opin. Struct. Biol (1996) 6(4), 527-33.) Synthetic ribozymes can be designed to specifically cleave CSB5 mRNAs at selected positions thereby preventing translation of the CSB5 mRNAs into functional polypeptide. Ribozymes may be synthesised with a natural ribose phosphate backbone and natural bases, as normally found in RNA molecules. Alternatively the ribosymes may be synthesised with non-natural backbones to provide protection from ribonuclease degradation, for example, 2'-O-methyl RNA, and may contain modified bases.

For treating abnormal conditions related to an under-expression of CSB5 and its activity, several approaches are also available. One approach comprises administering to a subject a therapeutically effective amount of a compound which activates a polypeptide of the present invention, i.e., an agonist as described above, in combination with a pharmaceutically acceptable carrier, to thereby alleviate the abnormal condition. Alternatively, gene therapy may be employed to effect the endogenous production of CSB5 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For an overview of gene therapy, see Chapter 20, Gene Therapy and other Molecular Genetic-based Therapeutic Approaches, (and references cited therein) in Human Molecular Genetics, T Strachan and A P Read, BIOS Scientific Publishers Ltd (1996). Another approach is to administer a therapeutic amount of a polypeptide of the present invention in combination with a suitable pharmaceutical carrier.

In a further aspect, the present invention provides for pharmaceutical compositions comprising a therapeutically effective amount of a polypeptide, such as the soluble form of a polypeptide of the present invention, agonist/antagonist peptide or small molecule compound, in combination with a pharmaceutically acceptable carrier or excipient. Such carriers include, but are not limited to, saline, buffered saline, dextrose, water, glycerol, ethanol, and combinations thereof. The invention further relates to pharmaceutical packs and kits comprising one or more containers filled with one or more of the ingredients of the aforementioned compositions of the invention. Polypeptides and other compounds of the present invention may be employed alone or in conjunction with other compounds, such as therapeutic compounds.

The composition will be adapted to the route of administration, for instance by a systemic or an oral route. Preferred forms of systemic administration include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if a polypeptide or other compounds of the present invention can be formulated in an enteric or an encapsulated formulation, oral administration may also be possible. Administration of these compounds may also be topical and/or localized, in the form of salves, pastes, gels, and the like.

The dosage range required depends on the choice of peptide or other compounds of the present invention, the route of administration, the nature of the formulation, the nature of the subject's condition, and the judgment of the attending practitioner. Suitable dosages, however, are in the range of 0.1-100 μg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

Polynucleotide and polypeptide sequences form a valuable information resource with which to identify further sequences of similar homology. This is most easily facilitated by storing the sequence in a computer readable medium and then using the stored data to search a sequence database using well known searching tools, such as those in the GCG and Lasergene software packages. Accordingly, in a further aspect, the present invention provides for a computer readable medium having stored thereon a polynucleotide comprising the sequences of SEQ ID NO: 1 or SEQ ID NO: 5 and/or a polypeptide sequence encoded thereby.

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993: Wold, F., Post-translational Protein Modifications: Perspectives and Prospects, pgs. 1-12 in Post-translational Covalent Modification of Proteins, B. C. Johnson, Ed., Academic Press, New York, 1983; Seifter *et al.,* "Analysis for protein modifications and nonprotein cofactors", Meth Enzymol (1990) 182: 626-646 and Rattan *et al.,* "Protein Synthesis: Post-translational Modifications and Aging", Ann NY Acad Sci (1992) 663 :48-62).

"Variant" refers to a polynucleotide or polypeptide that differs from a reference polynucleotide or polypeptide, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis. "Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. "Identity" and "similarity" can be readily calculated by known methods, including but not limited to those described in (Computational Molecular Biology, Lesk, A.M., ed., Oxford University Press, New York, 1988; Biocomputing: Informatics and Genome Projects, Smith, D.W., ed., Academic Press, New York, 1993; Computer Analysis of Sequence Data, Part I, Griffin, A.M., and Griffin, H.G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM *J. Applied Math., 48: 1073* (1988). Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research *12(1).* 387 (1984)), BLASTP, BLASTN, and FASTA (Atschul, S. F. et al., J. Molec. Biol. *215:* 403-410 (1990). The BLAST X program is publicly available from NCBI and other sources (BLAST Manual, Altschul, S., *et al.,* NCBI NLM NIH Bethesda, MD 20894; Altschul, S., *et al.,* J. Mol. Biol. *215:* 403-410 (1990). The well known Smith Waterman algorithm may also be used to determine identity.

Preferred parameters for polypeptide sequence comparison include the following:

1) Algorithm: Needleman and Wunsch, J. Mol Biol. 48: 443-453 (1970)
Comparison matrix: BLOSSUM62 from Hentikoff and Hentikoff, Proc. Natl. Acad. Sci. USA. 89:10915-10919 (1992)
Gap Penalty: 12
Gap Length Penalty: 4

A program useful with these parameters is publicly available as the "gap" program from Genetics Computer Group, Madison WI. The aforementioned parameters are the default parameters for polypeptide comparisons (along with no penalty for end gaps).

Preferred parameters for polynucleotide comparison include the following:

1) Algorithm: Needleman and Wunsch, J. Mol Biol. 48: 443-453 (1970)
Comparison matrix: matches = +10, mismatch = 0
Gap Penalty: 50
Gap Length Penalty: 3

A program useful with these parameters is publicly available as the "gap" program from Genetics Computer Group, Madison WI. The aforementioned parameters are the default parameters for polynucleotide comparisons.

By way of example, a polynucleotide sequence of the present invention may be identical to the reference sequences of SEQ ID NO: 1 or SEQ ID NO:5, that is be 100% identical, or it may include up to a certain integer number of nucleotide alterations as compared to the reference sequence. Such alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in SEQ ID NO:1 or SEQ ID NO:5 by the numerical percent of the respective percent identity (divided by 100) and subtracting that product from said total number of nucleotides in SEQ ID NO:1 or SEQ ID NO:5, or:

$$n_n \leq x_n - (x_n \bullet y),$$

wherein $\mathbf{n_n}$ is the number of nucleotide alterations, $\mathbf{x_n}$ is the total number of nucleotides in SEQ ID NO:1 or SEQ ID NO:5, and **y** is, for instance, 0.70 for 70%, 0.80 for 80%, 0.85 for 85%. 0.90 for 90%, 0.95 for 95%,etc., and wherein any non-integer product of $\mathbf{x_n}$ and **y** is rounded down to the nearest integer prior to subtracting it from $\mathbf{x_n}$. Alterations of a polynucleotide sequence encoding the polypeptide of SEQ ID NO:2 or SEQ ID NO:6 may create nonsense, missense or frameshift mutations in this coding sequence and thereby alter the polypeptide encoded by the polynucleotide following such alterations.

Similarly, a polypeptide sequence of the present invention may be identical to the reference sequences of SEQ ID NO:2 or SEQ ID NO:6, that is be 100% identical, or it may include up to a certain integer number of amino acid alterations as compared to the reference sequence such that the % identity is less than 100%. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 or SEQ ID NO:6 by the numerical percent of the respective percent identity(divided by 100) and then subtracting that product from said total number of amino acids in SEQ ID NO:2 or SEQ ID NO:6, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2 or SEQ ID NO:6, and **y** is, for instance 0.70 for 70%, 0.80 for 80%, 0.85 for 85% etc., and wherein any non-integer product of $\mathbf{x_a}$ and **y** is rounded down to the nearest integer prior to subtracting it from $\mathbf{x_a}$.

"Homolog" is a generic term used in the art to indicate a polynucleotide or polypeptide sequence possessing a high degree of sequence relatedness to a subject sequence. Such relatedness may be quantified by determining the degree of identity and/or similarity between the sequences being compared as hereinbefore described.

Falling within this generic term are the terms "ortholog", meaning a polynucleotide or polypeptide that is the functional equivalent of a polynucleotide or polypeptide in another species, and "paralog" meaning a functionally similar sequence when considered within the same species. Hence in the rat, for example, a member of the family of serotonin receptors is a paralog of the other members of the rat serotonin receptor family.

"Fusion protein" refers to a protein encoded by two, often unrelated, fused genes or fragments thereof. In one example, EP-A-0 464 discloses fusion proteins comprising various portions of constant region of immunoglobulin molecules together with another human protein or part thereof. In many cases, employing an immunoglobulin Fc region as a part of a fusion protein is advantageous for use in therapy and diagnosis resulting in, for example, improved pharmacokinetic properties [see, e.g., EP-A 0232 262]. On the other hand, for some uses it would be desirable to be able to delete the Fc part after the fusion protein has been expressed, detected and purified.

All publications, including but not limited to patents and patent applications, cited in this specification are herein incorporated by reference as if each individual publication were specifically and individually indicated to be incorporated by reference herein as though fully set forth.

## SEQUENCE INFORMATION

### SEQ ID NO:1

ACGAGCCCGGCGCCGTCCCCGCCCCGCCCCCCGTGATTCCCCCTGCATGGCCGGCCCGGGTGGGGGGCGCGG
GGGGGCCCGGGCGCCATGCGGGGGGGGCACAAAGGGGGTCGCTGTGCCTGTCCCCGTGTGATCCGAAAAGTG
CTGGCAAAATGCGGCTGCTGCTTCGCCCGGGGGGGACGTGAATCCTATTCCATTGCGGGCAGTGAGGGGAGT
ATATCGGCTTCTGCTGCCTCCGGTCTGGCTGCCCCCTCTGGCCCCAGCTCTGGCCTCAGCTCTGGCCCCTGT
TCCCCAGGCCCCCCAGGGCCCGTCAGTGGCCTGAGGAGATGGTTGGATCATTCCAAACATTGTCTCAGTGTG
GAAACTGAGGCAGACAGTGGTCAGGCAGGACCATATGAGAACTGGATGTTGGAGCCAGCTCTAGCCACAGGA
GAGGAGCTGCCGGAACTGACCTTGCTGACCACACTGTTGGAAGGCCCTGGAGATAAGACGCAGCCACCTGAA
GAGGAGACTTTGTCCCAAGCCCCTGAGAGTGAGGAGGAACAGAAGAAGAAGGCTCTGGAAAGGAGTATGTAT
GTCCTGAGTGAACTGGTAGAAACAGAGAAAATGTACGTTGACGACTTGGGGCAGATTGTGGAGGGTTATATG
GCCACCATGGCTGCTCAGGGGGTCCCCGAGAGTCTTCGAGGCCGTGACAGGATTGTGTTTGGGAATATCCAG
CAAATCTATGAGTGGCACCGAGACTATTTCTTGCAAGAGCTACAACGGTGTCTGAAAGATCCTGATTGGCTG
GCTCAGCTATTCATCAAACACGAGCGCCGGCTGCATATGTATGTGGTGTACTGTCAGAATAAGCCCAAGTCA
GAGCATGTGGTGTCAGAGTTTGGGGACAGCTACTTTGAGGAGCTCCGGCAGCAGCTGGGGCACCGCCTGCAG
CTGAACGACCTCCTCATCAAACCTGTGCAGCGGATCATGAAATACCAGCTGCTGCTCAAGGATTTTCTCAAG
TATTACAATAGAGCTGGGATGGATACTGCAGACCTAGAGCAAGCTGTGGAGGTCATGTGCTTTGTGCCCAAG
CGCTGCAACGATATGATGACGCTGGGGAGATTGCGGGGATTTGAGGGCAAACTGACTGCTCAGGGGAAGCTC
TTGGGCCAGGACACTTTCTGGGTCACCGAGCCTGAGGCTGGAGGGCTGCTGTCTTCCCGAGGTCGAGAGAGG
CGCGTCTTCCTCTTTGAGCAAATCATCATCTTCAGTGAAGCCCTGGGAGGAGGAGTGAGAGGTGGAACACAG
CCTGGATATGTATACAAGAACAGCATTAAGGTGAGCTGCCTGGGACTGGAGGGGAACCTCCAAGGTGACCCT
TGCCGCTTTGCACTGACCTCCAGAGGGCCAGAGGGTGGGATCCAGCGCTATGTCCTGCAGGCTGCAGACCCT
GCTATCAGTCAGGCCTGGATCAAGCATGTGGCTCAGATCTTGGAGAGCCAACGGGACTTCCTCAACGCATTG
CAGTCACCCATTGAGTACCAGAGACGGGAGAGCCAGACCAACAGCCTGGGGCGGCCAAGAGGGCCTGGAGTG
GGGAGCCCTGGAAGAATTCGGCTTGGAGATCAGGCCCAGGGCAGCACACACACACCCATCAATGGCTCTCTC
CCCTCTCTGCTGCTGTCACCCAAAGGGGAGGTGGCCAGAGCCCTCTTGCCACTGGATAAACAGGCCCTTGGT
GACATCCCCCAGGCTCCCCATGACTCTCCTCCAGTCTCTCCAACTCCAAAAACCCCTCCCTGCCAAGCCAGA
CTTGCCAAGCTGGATGAAGATGAGCTGTAACTGGTGAAAACCATGGGGGTGGTGCTGACTCAGCCGCCTATT
CCCCAAGGAGCTTCAGGGCAGTCCTTCTGGCACTGCTCCAGAATTCCTCCTTCTTGGTGTGTCTGGAGGGTG
GGCAAGGCTGGGAGGGATATCAACTTGGAGGAGAACACCTAGACCCAAGGACTTTTTTCTGCCCAAGGAACA
CAGTTTCCTTCAGCTCCCATCCCTATGCATGCATCATGGTCCCCCCAAAAGGAGGATATGTGGGTGGGTGGG
AGGGCTGGGGCAGGGGCCAGATAGAAATTATTGGTTTTGTTTTTTAATTTTGTTTTTCCTGTTTTCTGAGAA
TAAAGGTTTTGTTATATC

### SEQ ID NO:2

MRGGHKGGRCACPRVIRKVLAKCGCCFARGGRESYSIAGSEGSISASAASGLAAPSGPSSGLSSGPCSPGPP
GPVSGLRRWLDHSKHCLSVETEADSGQAGPYENWMLEPALATGEELPELTLLTTLLEGPGDKTQPPEEETLS
QAPESEEEQKKALERSMYVLSELVETEKMYVDDLGQIVEGYMATMAAQGVPESLRGRDRIVFGNIQQIYEW
HRDYFLQELQRCLKDPDWLAQLFIKHERRLHMYVVYCQNKPKSEHVVSEFGDSYFEELRQQLGHRLQLNDLL
IKPVQRIMKYQLLLKDFLKYYNRAGMDTADLEQAVEVMCFVPKRCNDMMTLGRLRGFEGKLTAQGKLLGQDT
FWVTEPEAGGLLSSRGRERRVFLFEQIIIFSEALGGGVRGGTQPGYVYKNSIKVSCLGLEGNLQGDPCRFAL
TSRGPEGGIQRYVLQAADPAISQAWIKHVAQILESQRDFLNALQSPIEYQRRESQTNSLGRPRGPGVGSPGR
IRLGDQAQGSTHTPINGSLPSLLLSPKGEVARALLPLDKQALGDIPQAPHDSPPVSPTPKTPPCQARLAKLD
EDEL

### SEQ ID NO:3

GACTT GGGG CAGATT GT GGAGGGT T AT AT GG CCACCAT GG CT G CT CAGGGGGT CCCCGAG
AGT CTT CGAGG CCGT GACAGGATT GT GTTT GGGAAT AT CCAG CAAAT CT AT GAGT GG CAC
CGAGACT AT TT CTT G CAAGAG CT ACAACGGT GT CT GAAAGAT CCT GATT GG CT GG CT CAG
CT ATT CAT CAAACACGAG CG CCGG CT G CAT AT GT AT GT GGT GT ACT GT CAGAAT AAG CCC
AAGT CAGAG CAT GT GGT GT CAGAGTTT GGGGACAG CT ACTTT GAGGAG CT CCGG CAG CAG
CT GGGG CACCG CCT G CAG CT GAACGACCT CCT CAT CAAACCT GT G CAG CGGAT CAT GAAA
T ACCAG CT G CT G CT CAAGGATTTT CT CAAGT ATT ACAAT AGAG CT GGGAT GGAT ACT G CA
GACCT AGAG CAAG CT GT GGAGGT CAT GT G CTTT GT G CCCAAG CG CT G CAACGAT AT GAT G
ACG CT GGGGAGATT G CGGGGATTT GAGGG CAAACT GACT G CT CAGGGGAAG CT CTT GGG C
CAGGACACTTT CT GGGT CACCGAG CCT GAGG CT GGAGGG CT G CT GTTTT CCCGAGGT CGA
GAGAGG CG CGTTTT CCT CTTT GAG CAAAT CAT CAT CTT CAGT GAAG CCCT GGGAGGAGGA
GT NAGAGGT GGAACAAAG CCT GGAT AT GT ATT ACAAGACCAG CAT

**SEQ ID NO:4**

D LGQI VEGY MAT MAAQGVP E SL RG RD RI VFGNI QQI YEWH RD YFLQELQ RCLKDPDWLAQ
LF IKHE RRLHMYVVY CQ NKPK SEHVV SEFGD SYFEEL RQQLGH RLQL ND LL IKPVQ RFMK
YQLLLKD FLKYYN RAGMDT AD LEQAVEVMCFVPK RCND MMT LG RL RG FEGKLT AQGKLLG
QDT FWVT EP EAGGLLF S RG RE RRVFLFEQI I I F SEALGGGV RGGTKP GYVLQDQH

**SEQ ID NO:5**

ACGAG CCCGG CG CCGT CCCCG CCCCG CCCCCCGT GATT CCCCCT G CAT GG CCGG CCCGGGT GGGGGG CG CGG
GGGGG CCCGGG CG CCAT G CGGGGGGGG CACAAAGGGGT CG CT GT G CCT GT CCCCGT GT GAT CCGAAAAGT G
CT GG CAAAAT G CGG CT G CT G CTT CG CCCGGGGGGGACGT GAAT CCT ATT CCATT G CGGG CAGT GAGGGGAGT
AT AT CGG CTT CT G CT G CCT CCGGT CT GG CT G CCCCCT CT GG CCCCAG CT CT GG CCT CAG CT CT GG CCCCT GT
T CCCCAGG CCCCCCAGGG CCCGT CAGT GG CCT GAGGAGAT GGTT GGAT CATT CCAAACATT GT CT CAGT GT G
GAAACT GAGG CAGACAGT GGT CAGG CAGGACCAT AT GAGAACT GGAT GTT GGAG CCAG CT CT AG CCACAGGA
GAGGAG CT G CCGGAACT GACCTT G CT GACCACACT GTT GGAGGG CCCT GGAGAT AAGACG CAG CCACCT GAA
GAGGAGACTTT GT CCCAAG CCCCT GAGAGT GAGGAGGAACAGAAGAAGAAGG CT CT GGAAAGGAGT AT GT AT
GT CCT GAGT GAACT GGT AGAAACAGAGAAAAT GT ACGT GGACGACTT GGGG CAGATT GT GGAGGGTT AT AT G
G CCACCAT GG CT G CT CAGGGGGT CCCCGAGAGT CTT CGAGG CCGT GACAGGATT GT GTTT GGGAAT AT CCAG
CAAAT CT AT GAGT GG CACCGAGACT ATTT CTT G CAAGAG CT ACAACGGT GT CT GAAAGAT CCT GATT GG CT G
G CT CAG CT ATT CAT CAAACACGAG CT CCGG CAG CAG CT GGGG CACCG CCT G CAG CT GAACGACCT CCT CAT C
AAACCT GT G CAG CGGAT CAT GAAAT ACCAG CT G CT G CT CAAGGATTTT CT CAAGT ATT ACAAT AGAG CT GGG
AT GGAT ACT G CAGACCT AGAG CAAG CT GT GGAGGT CAT GT G CTTT GT G CCCAAG CG CT G CAACGAT AT GAT G
ACG CT GGGGAGATT G CGGGGATTT GAGGG CAAACT GACT G CT CAGGGGAAG CT CTT GGG CCAGGACACTTT C
T GGGT CACCGAG CCT GAGG CT GGAGGG CT G CT GT CTT CCCGAGGT CGAGAGAGG CG CGT CTT CCT CTTT GAG
CAAAT CAT CAT CTT CAGT GAAG CCCT GGGAGGAGGAGT GAGAGGT GGAACACAG CCT GGAT AT GT AT ACAAG
AACAG CATT AAGGT GAG CT G CCT GGGACT GGAGGGGAACCT CCAAGGT GACCCTT G CCG CTTT G CACT GACC
T CCAGAGGG CCAGAGGGT GGGAT CCAG CG CT AT GT CCT G CAGG CT G CAGACCCT G CT AT CAGT CAGG CCT GG

ATCAAGCATGTGGCTCAGATCTTGGAGAGCCAACGGGACTTCCTCAACGCATTGCAGTCACCCATTGAGTAC
CAGAGACGGGAGAGCCAGACCAACAGCCTGGGGCGGCCAAGAGGGCCTGGAGTGGGGAGCCCTGGAAGAATT
CGGCTTGGAGATCAGGCCCAGGGCAGCACACACACACCCATCAATGGCTCTCTCCCCTCTCTGCTGCTGTCA
CCCAAAGGGGAGGTGGCCAGAGCCCTCTTGCCACTGGATAAACAGGCCCTTGGTGACATCCCCCAGGCTCCC
CATGACTCTCCTCCAGTCTCTCCAACTCCAAAAACCCCTCCCTGCCAAGCCAGACTTGCCAAGCTGGATGAA
GATGAGCTGTAACTGGTGAAAACCATGGGGGTGGTGCTGACTCAGCCGCCTATTCCCCAAGGAGCTTCAGGG
CAGTCCTTCTGGCACTGCTCCAGAATTCCTCCTTCTTGGTGTGTCTGGAGGGTGGGCAAGGCTGGGAGGGAT
ATCAACTTGGAGGAGAACACCTAGACCCAAGGACTTTTTTCTGCCCAAGGAACACAGTTTCCTTCAGCTCCC
ATCCCTATGCATGCATCATGGTCCCCCCAAAAGGAGGATATGTGGGTGGGTGGGAGGGCTGGGGCAGGGGCC
AGATAGAAATTATTGGTTTTGTTTTTTAATTTTGTTTTTCCTGTTTTCTGAGAATAAAGGTTTTGTTATATC

**SEQ ID NO:6**

MRGGHKGGRCACPRVIRKVLAKCGCCFARGGRESYSIAGSEGSISASAASGLAAPSGPSSGLSSGPCSPGPP
GPVSGLRRWLDHSKHCLSVETEADSGQAGPYENWMLEPALATGEELPELTLLTTLLEGPGDKTQPPEEETLS
QAPESEEEQKKKALERSMYVLSELVETEKMYVDDLGQIVEGYMATMAAQGVPESLRGRDRIVFGNIQQIYEW
HRDYFLQELQRCLKDPDWLAQLFIKHELRQQLGHRLQLNDLLIKPVQRIMKYQLLLKDFLKYYNRAGMDTAD
LEQAVEVMCFVPKRCNDMMTLGRLRGFEGKLTAQGKLLGQDTFWVTEPEAGGLLSSRGRERRVFLFEQIIIF
SEALGGGVRGGTQPGYVYKNSIKVSCLGLEGNLQGDPCRFALTSRGPEGGIQRYVLQAADPAISQAWIKHVA
QILESQRDFLNALQSPIEYQRRESQTNSLGRPRGPGVGSPGRIRLGDQAQGSTHTPINGSLPSLLLSPKGEV
ARALLPLDKQALGDIPQAPHDSPPVSPTPKTPPCQARLAKLDEDEL

**Claims**

1. An isolated polypeptide selected from:

   (a) a polypeptide comprising an amino acid sequence which has at least 70% identity to the amino acid sequence of SEQ ID NO:2 and
   (b) a polypeptide comprising an amino acid sequence which has at least 70% identity to the amino acid sequence of SEQ ID NO:6.

2. An isolated polypeptide as claimed in claim I in which the amino acid sequence has at least 95% identity.

3. The polypeptide as claimed in claim 1 comprising the amino acid sequence of SEQ ID NO:2 or SEQ ID NO:6.

4. The isolated polypeptide of SEQ ID NO:2 or SEQ ID NO:6.

5. An isolated polynucleotide selected from:

   (a) a polynucleotide comprising a nucleotide sequence encoding a polypeptide that has at least 70% identity to the amino acid sequence of SEQ ID NO:2 over the entire length of SEQ ID NO:2, and
   (b) a polynucleotide comprising a nucleotide sequence encoding a polypeptide that has at least 70% identity to the amino acid sequence of SEQ ID NO:6 over the entire length of SEQ ID NO:6, or a nucleotide sequence complementary to said isolated polynucleotide.

6. An isolated polynucleotide selected from:

   (a) a polynucleotide comprising a nucleotide sequence that has at least 70% identity to a nucleotide sequence encoding a polypeptide of SEQ ID NO:2 over the entire coding region, and
   (b) a polynucleotide comprising a nucleotide sequence that has at least 70% identity to a nucleotide sequence encoding a polypeptide of SEQ ID NO:6, over the entire coding region, or a nucleotide sequence complementary to said isolated polynucleotide.

7. An isolated polynucleotide selected from:

   (a) a polynucleotide which comprises a nucleotide sequence which has at least 70% identity to that of SEQ ID NO:1 over the entire length of SEQ ID NO: 1, and
   (b) a polynucleotide which comprises a nucleotide sequence which has at least 70% identity to that of SEQ ID NO:5 over the entire length of SEQ ID NO:5,

   or a nucleotide sequence complementary to said isolated polynucleotide.

8. The isolated polynucleotide as claimed in any one of claims 5 to 7 in which the identity is at least 95%.

9. An isolated polynucleotide selected from:

   (a) a polynucleotide comprising a nucleotide sequence encoding the polypeptide of SEQ ID NO:2,
   (b) the polynucleotide of SEQ ID NO:1;
   (c) a polynucleotide obtainable by screening an appropriate library under stringent hybridization conditions with a labeled probe having the sequence of SEQ ID NO: 1 or a fragment thereof, or a nucleotide sequence complementary to said isolated polynucleotide.

10. An isolated polynucleotide selected from:

   (a) a polynucleotide comprising a nucleotide sequence encoding the polypeptide of SEQ ID NO:6,
   (b) the polynucleotide of SEQ ID NO:5;
   (c) a polynucleotide obtainable by screening an appropriate library under stringent hybridization conditions with a labeled probe having the sequence of SEQ ID NO:5 or a fragment thereof,

   or a nucleotide sequence complementary to said isolated polynucleotide.

**11.** An expression system comprising a polynucleotide capable of producing a polypeptide of claim 1 when said expression system is present in a compatible host cell.

**12.** A host cell comprising the expression system of claim 11 or a membrane thereof expressing the polypeptide of claim 1.

**13.** A process for producing a polypeptide of claim 1 comprising culturing a host cell of claim 12 under conditions sufficient for the production of said polypeptide and recovering the polypeptide from the culture medium.

**14.** An antibody immunospecific for the polypeptide of claim 1.

**15.** A method for screening to identify compounds which stimulate or which inhibit the function of the polypeptide of claim 1 which comprises a method selected from the group consisting of:

(a) measuring the binding of a candidate compound to the polypeptide (or to the cells or membranes bearing the polypeptide) or a fusion protein thereof by means of a label directly or indirectly associated with the candidate compound;
(b) measuring the binding of a candidate compound to the polypeptide (or to the cells or membranes bearing the polypeptide) or a fusion protein thereof in the presense of a labeled competitior;
(c) testing whether the candidate compound results in a signal generated by activation or inhibition of the polypeptide, using detection systems appropriate to the cells or cell membranes bearing the polypeptide;
(d) mixing a candidate compound with a solution containing a polypeptide of claim 1, to form a mixture, measuring activity of the polypeptide in the mixture, and comparing the activity of the mixture to a standard; or
(e) detecting the effect of a candidate compound on the production of mRNA encoding said polypeptide and said polypeptide in cells, using for instance, an ELISA assay.

**16.** An agonist or antagonist to the polypeptide of claims 1 to 4.

**17.** A compound which is:

(a) an agonist or antagonist to the polypeptide of claims I to 4;
(b) isolated polynucleotide of claims 5 to 10; or
(c) a nucleic acid molecule that modulates the expression of the nucleotide sequence encoding the polypeptide of claim 1;

for use in therapy.

**18.** A process for diagnosing a disease or a susceptibility to a disease in a subject related to expression or activity of the polypeptide of claim 1 in a subject comprising:

(a) determining the presence or absence of a mutation in the nucleotide sequence encoding said polypeptide in the genome of said subject; and/or
(b) analyzing for the presence or amount of said polypeptide expression in a sample derived from said subject.

**19.** An isolated polynucleotide selected form the group consisting of:

(a) an isolated polynucleotide comprising a nucleotide sequence which has at least 75% identity to SEQ ID NO:3 over the entire length of SEQ ID NO:3;
(b) an isolated polynucleotide comprising the polynucleotide of SEQ ID NO:3;
(c) the polynucleotide of SEQ ID NO:3; or
(d) an isolated polynucleotide comprising a nucleotide sequence encoding a polypeptide which has at least 70% identity to the amino acid sequence of SEQ ID NO:4, over the entire length of SEQ ID NO:4.

**20.** A polypeptide selected from the group consisting of:

(a) a polypeptide which comprises an amino acid sequence which has at least 70% identity to that of SEQ ID NO:4 over the entire length of SEQ ID NO:4;
(b) a polypeptide in which the amino acid sequence has at least 70% identity to the amino acid sequence of SEQ ID NO:4 over the entire length of SEQ ID NO:4;
(c) a polypeptide which comprises the amino acid of SEQ ID NO:4;
(d) a polypeptide which is the polypeptide of SEQ ID NO:4; or
(e) a polypeptide which is encoded by a polynucleotide comprising the sequence contained in SEQ ID NO:3.

**21.** An isolated polynucleotide selected form the group consisting of:

(a) an isolated polynucleotide comprising a nucleotide sequence which has at least 70% identity to SEQ ID NO:5 over the entire length of SEQ ID NO:5;
(b) an isolated polynucleotide comprising the polynucleotide of SEQ ID NO:5;
(c) the polynucleotide of SEQ ID NO:5; or
(d) an isolated polynucleotide comprising a nucleotide sequence encoding a polypeptide which has at least 70% identity to the amino acid sequence of SEQ ID NO:6, over the entire length of SEQ ID NO:6.

**22.** A polypeptide selected from the group consisting of:

(a) a polypeptide which comprises an amino acid sequence which has at least 70% identity to that of SEQ ID NO:6 over the entire length of SEQ ID NO:6;
(b) a polypeptide in which the amino acid sequence has at least 70% identity to the amino acid sequence of SEQ ID NO:6 over the entire length of SEQ ID NO:6;
(c) a polypeptide which comprises the amino acid of SEQ ID NO:6:
(d) a polypeptide which is the polypeptide of SEQ ID NO:6; or
(e) a polypeptide which is encoded by a polynucleotide comprising the sequence contained in SEQ ID NO:5.

**Annex to the description**

SEQUENCE LISTING

(1) GENERAL INFORMATION

(I) APPLICANT: SMITHKLINE BEECHAM LABORATOIRES PHARMACEUTIQUES AND SMITHKLINE BEECHAM CORPORATION

(II) TITLE OF THE INVENTION: NOVEL COMPOUNDS

(III) NUMBER OF SEQUENCES: 6

(IV) CORRESPONDENCE ADDRESS:
(A) ADDRESSEE: SMITHKLINE BEECHAM, CORPORATE INTELLECTUAL PROPERTY
(B) STREET: TWO NEW HORIZONS COURT
(C) CITY: BRENTFORD
(D) STATE: MIDDLESEX
(E) COUNTRY: UK
(F) ZIP: TW8 9EP

(V) COMPUTER READABLE FORM:
(A) MEDIUM TYPE: DISKETTE
(B) COMPUTER: IBM COMPATIBLE
(C) OPERATING SYSTEM: DOS
(D) SOFTWARE: FASTSEQ FOR WINDOWS VERSION 2.0

(2) INFORMATION FOR SEQ ID NO:1:

(I) SEQUENCE CHARACTERISTICS:
(A) LENGTH: 2178 BASE PAIRS
(B) TYPE: NUCLEIC ACID
(C) STRANDEDNESS: SINGLE
(D) TOPOLOGY: LINEAR

(II) MOLECULE TYPE: CDNA

(XI) SEQUENCE DESCRIPTION: SEQ ID NO: 1:

```
ACGAGCCCGG CGCCGTCCCC GCCCCGCCCC CCGTGATTCC CCCTGCATGG CCGGCCCGGG   60
TGGGGGGCGC GGGGGGGCCC GGGCGCCATG CGGGGGGGGC ACAAAGGGGG TCGCTGTGCC  120
TGTCCCCGTG TGATCCGAAA AGTGCTGGCA AAATGCGGCT GCTGCTTCGC CCGGGGGGGA  180
CGTGAATCCT ATTCCATTGC GGGCAGTGAG GGGAGTATAT CGGCTTCTGC TGCCTCCGGT  240
CTGGCTGCCC CCTCTGGCCC CAGCTCTGGC CTCAGCTCTG GCCCCTGTTC CCCAGGCCCC  300
CCAGGGCCCG TCAGTGGCCT GAGGAGATGG TTGGATCATT CCAAACATTG TCTCAGTGTG  360
GAAACTGAGG CAGACAGTGG TCAGGCAGGA CCATATGAGA ACTGGATGTT GGAGCCAGCT  420
CTAGCCACAG GAGAGGAGCT GCCGGAACTG ACCTTGCTGA CCACACTGTT GGAAGGCCCT  480
GGAGATAAGA CGCAGCCACC TGAAGAGGAG ACTTTGTCCC AAGCCCCTGA GAGTGAGGAG  540
GAACAGAAGA AGAAGGCTCT GGAAAGGAGT ATGTATGTCC TGAGTGAACT GGTAGAAACA  600
GAGAAAATGT ACGTTGACGA CTTGGGGCAG ATTGTGGAGG GTTATATGGC CACCATGGCT  660
GCTCAGGGGG TCCCCGAGAG TCTTCGAGGC CGTGACAGGA TTGTGTTTGG GAATATCCAG  720
CAAATCTATG AGTGGCACCG AGACTATTTC TTGCAAGAGC TACAACGGTG TCTGAAAGAT  780
CCTGATTGGC TGGCTCAGCT ATTCATCAAA CACGAGCGCC GGCTGCATAT GTATGTGGTG  840
TACTGTCAGA ATAAGCCCAA GTCAGAGCAT GTGGTGTCAG AGTTTGGGGA CAGCTACTTT  900
GAGGAGCTCC GGCAGCAGCT GGGGCACCGC CTGCAGCTGA ACGACCTCCT CATCAAACCT  960
GTGCAGCGGA TCATGAAATA CCAGCTGCTG CTCAAGGATT TTCTCAAGTA TTACAATAGA 1020
GCTGGGATGG ATACTGCAGA CCTAGAGCAA GCTGTGGAGG TCATGTGCTT TGTGCCCAAG 1080
CGCTGCAACG ATATGATGAC GCTGGGGAGA TTGCGGGGAT TTGAGGGCAA ACTGACTGCT 1140
CAGGGGAAGC TCTTGGGCCA GGACACTTTC TGGGTCACCG AGCCTGAGGC TGGAGGGCTG 1200
CTGTCTTCCC GAGGTCGAGA GAGGCGCGTC TTCCTCTTTG AGCAAATCAT CATCTTCAGT 1260
GAAGCCCTGG GAGGAGGAGT GAGAGGTGGA ACACAGCCTG GATATGTATA CAAGAACAGC 1320
ATTAAGGTGA GCTGCCTGGG ACTGGAGGGG AACCTCCAAG GTGACCCTTG CCGCTTTGCA 1380
CTGACCTCCA GAGGGCCAGA GGGTGGGATC CAGCGCTATG TCCTGCAGGC TGCAGACCCT 1440
GCTATCAGTC AGGCCTGGAT CAAGCATGTG GCTCAGATCT TGGAGAGCCA ACGGGACTTC 1500
CTCAACGCAT TGCAGTCACC CATTGAGTAC CAGAGACGGG AGAGCCAGAC CAACAGCCTG 1560
GGGCGGCCAA GAGGGCCTGG AGTGGGGAGC CCTGGAAGAA TTCGGCTTGG AGATCAGGCC 1620
CAGGGCAGCA CACACACACC CATCAATGGC TCTCTCCCCT CTCTGCTGCT GTCACCCAAA 1680
GGGGAGGTGG CCAGAGCCCT CTTGCCACTG GATAAACAGG CCCTTGGTGA CATCCCCCAG 1740
GCTCCCCATG ACTCTCCTCC AGTCTCTCCA ACTCCAAAAA CCCCTCCCTG CCAAGCCAGA 1800
CTTGCCAAGC TGGATGAAGA TGAGCTGTAA CTGGTGAAAA CCATGGGGGT GGTGCTGACT 1860
CAGCCGCCTA TTCCCCAAGG AGCTTCAGGG CAGTCCTTCT GGCACTGCTC CAGAATTCCT 1920
CCTTCTTGGT GTGTCTGGAG GGTGGGCAAG GCTGGGAGGG ATATCAACTT GGAGGAGAAC 1980
ACCTAGACCC AAGGACTTTT TTCTGCCCAA GGAACACAGT TTCCTTCAGC TCCCATCCCT 2040
ATGCATGCAT CATGGTCCCC CCAAAAGGAG GATATGTGGG TGGGTGGGAG GGCTGGGGCA 2100
GGGGCCAGAT AGAAATTATT GGTTTTGTTT TTTAATTTTG TTTTTCCTGT TTTCTGAGAA 2160
```

TAAAGGTTTT GTTATATC 2178

(2) INFORMATION FOR SEQ ID NO: 2:

(I) SEQUENCE CHARACTERISTICS:

(A) LENGTH: 580 AMINO ACIDS

(B) TYPE: AMINO ACID

(C) STRANDEDNESS: SINGLE

(D) TOPOLOGY: LINEAR

(II) MOLECULE TYPE: PROTEIN

(XI) SEQUENCE DESCRIPTION: SEQ ID NO: 2:

```
MET ARG GLY GLY HIS LYS GLY GLY ARG CYS ALA CYS PRO ARG VAL ILE
 1               5                  10                  15
ARG LYS VAL LEU ALA LYS CYS GLY CYS CYS PHE ALA ARG GLY GLY ARG
            20                  25                  30
GLU SER TYR SER ILE ALA GLY SER GLU GLY SER ILE SER ALA SER ALA
        35                  40                  45
ALA SER GLY LEU ALA ALA PRO SER GLY PRO SER SER GLY LEU SER SER
    50                  55                  60
GLY PRO CYS SER PRO GLY PRO PRO GLY PRO VAL SER GLY LEU ARG ARG
65                  70                  75                  80
TRP LEU ASP HIS SER LYS HIS CYS LEU SER VAL GLU THR GLU ALA ASP
                85                  90                  95
SER GLY GLN ALA GLY PRO TYR GLU ASN TRP MET LEU GLU PRO ALA LEU
            100                 105                 110
ALA THR GLY GLU GLU LEU PRO GLU LEU THR LEU LEU THR THR LEU LEU
        115                 120                 125
GLU GLY PRO GLY ASP LYS THR GLN PRO PRO GLU GLU GLU THR LEU SER
    130                 135                 140
GLN ALA PRO GLU SER GLU GLU GLU GLN LYS LYS LYS ALA LEU GLU ARG
145                 150                 155                 160
SER MET TYR VAL LEU SER GLU LEU VAL GLU THR GLU LYS MET TYR VAL
                165                 170                 175
ASP ASP LEU GLY GLN ILE VAL GLU GLY TYR MET ALA THR MET ALA ALA
            180                 185                 190
GLN GLY VAL PRO GLU SER LEU ARG GLY ARG ASP ARG ILE VAL PHE GLY
        195                 200                 205
ASN ILE GLN GLN ILE TYR GLU TRP HIS ARG ASP TYR PHE LEU GLN GLU
```

210 215 220

LEU GLN ARG CYS LEU LYS ASP PRO ASP TRP LEU ALA GLN LEU PHE ILE
225 230 235 240

LYS HIS GLU ARG ARG LEU HIS MET TYR VAL VAL TYR CYS GLN ASN LYS
245 250 255

PRO LYS SER GLU HIS VAL VAL SER GLU PHE GLY ASP SER TYR PHE GLU
260 265 270

GLU LEU ARG GLN GLN LEU GLY HIS ARG LEU GLN LEU ASN ASP LEU LEU
275 280 285

ILE LYS PRO VAL GLN ARG ILE MET LYS TYR GLN LEU LEU LEU LYS ASP
290 295 300

PHE LEU LYS TYR TYR ASN ARG ALA GLY MET ASP THR ALA ASP LEU GLU
305 310 315 320

GLN ALA VAL GLU VAL MET CYS PHE VAL PRO LYS ARG CYS ASN ASP MET
325 330 335

MET THR LEU GLY ARG LEU ARG GLY PHE GLU GLY LYS LEU THR ALA GLN
340 345 350

GLY LYS LEU LEU GLY GLN ASP THR PHE TRP VAL THR GLU PRO GLU ALA
355 360 365

GLY GLY LEU LEU SER SER ARG GLY ARG GLU ARG ARG VAL PHE LEU PHE
370 375 380

GLU GLN ILE ILE ILE PHE SER GLU ALA LEU GLY GLY GLY VAL ARG GLY
385 390 395 400

GLY THR GLN PRO GLY TYR VAL TYR LYS ASN SER ILE LYS VAL SER CYS
405 410 415

LEU GLY LEU GLU GLY ASN LEU GLN GLY ASP PRO CYS ARG PHE ALA LEU
420 425 430

THR SER ARG GLY PRO GLU GLY GLY ILE GLN ARG TYR VAL LEU GLN ALA
435 440 445

ALA ASP PRO ALA ILE SER GLN ALA TRP ILE LYS HIS VAL ALA GLN ILE
450 455 460

LEU GLU SER GLN ARG ASP PHE LEU ASN ALA LEU GLN SER PRO ILE GLU
465 470 475 480

TYR GLN ARG ARG GLU SER GLN THR ASN SER LEU GLY ARG PRO ARG GLY
485 490 495

PRO GLY VAL GLY SER PRO GLY ARG ILE ARG LEU GLY ASP GLN ALA GLN
500 505 510

GLY SER THR HIS THR PRO ILE ASN GLY SER LEU PRO SER LEU LEU LEU
515 520 525

SER PRO LYS GLY GLU VAL ALA ARG ALA LEU LEU PRO LEU ASP LYS GLN
530 535 540

ALA LEU GLY ASP ILE PRO GLN ALA PRO HIS ASP SER PRO PRO VAL SER
545 550 555 560
PRO THR PRO LYS THR PRO PRO CYS GLN ALA ARG LEU ALA LYS LEU ASP
565 570 575
GLU ASP GLU LEU
580

(2) INFORMATION FOR SEQ ID NO: 3:

(I) SEQUENCE CHARACTERISTICS:
(A) LENGTH: 705 BASE PAIRS
(B) TYPE: NUCLEIC ACID
(C) STRANDEDNESS: SINGLE
(D) TOPOLOGY: LINEAR

(II) MOLECULE TYPE: CDNA

(XI) SEQUENCE DESCRIPTION: SEQ ID NO: 3:

GACTTGGGGC AGATTGTGGA GGGTTATATG GCCACCATGG CTGCTCAGGG GGTCCCCGAG 60
AGTCTTCGAG GCCGTGACAG GATTGTGTTT GGGAATATCC AGCAAATCTA TGAGTGGCAC 120
CGAGACTATT TCTTGCAAGA GCTACAACGG TGTCTGAAAG ATCCTGATTG GCTGGCTCAG 180
CTATTCATCA AACACGAGCG CCGGCTGCAT ATGTATGTGG TGTACTGTCA GAATAAGCCC 240
AAGTCAGAGC ATGTGGTGTC AGAGTTTGGG GACAGCTACT TTGAGGAGCT CCGGCAGCAG 300
CTGGGGCACC GCCTGCAGCT GAACGACCTC CTCATCAAAC CTGTGCAGCG GATCATGAAA 360
TACCAGCTGC TGCTCAAGGA TTTTCTCAAG TATTACAATA GAGCTGGGAT GGATACTGCA 420
GACCTAGAGC AAGCTGTGGA GGTCATGTGC TTTGTGCCCA AGCGCTGCAA CGATATGATG 480
ACGCTGGGGA GATTGCGGGG ATTTGAGGGC AAACTGACTG CTCAGGGGAA GCTCTTGGGC 540
CAGGACACTT TCTGGGTCAC CGAGCCTGAG GCTGGAGGGC TGCTGTTTTC CCGAGGTCGA 600
GAGAGGCGCG TTTTCCTCTT TGAGCAAATC ATCATCTTCA GTGAAGCCCT GGGAGGAGGA 660
GTNAGAGGTG GAACAAAGCC TGGATATGTA TTACAAGACC AGCAT 705

(2) INFORMATION FOR SEQ ID NO: 4:

(I) SEQUENCE CHARACTERISTICS:
(A) LENGTH: 235 AMINO ACIDS
(B) TYPE: AMINO ACID
(C) STRANDEDNESS: SINGLE
(D) TOPOLOGY: LINEAR

(II) MOLECULE TYPE: PROTEIN

(XI) SEQUENCE DESCRIPTION: SEQ ID NO: 4:

```
ASP LEU GLY GLN ILE VAL GLU GLY TYR MET ALA THR MET ALA ALA GLN
 1              5                   10                  15
GLY VAL PRO GLU SER LEU ARG GLY ARG ASP ARG ILE VAL PHE GLY ASN
            20                  25                  30
ILE GLN GLN ILE TYR GLU TRP HIS ARG ASP TYR PHE LEU GLN GLU LEU
        35                  40                  45
GLN ARG CYS LEU LYS ASP PRO ASP TRP LEU ALA GLN LEU PHE ILE LYS
    50                  55                  60
HIS GLU ARG ARG LEU HIS MET TYR VAL VAL TYR CYS GLN ASN LYS PRO
65                  70                  75                  80
LYS SER GLU HIS VAL VAL SER GLU PHE GLY ASP SER TYR PHE GLU GLU
                85                  90                  95
LEU ARG GLN GLN LEU GLY HIS ARG LEU GLN LEU ASN ASP LEU LEU ILE
            100                 105                 110
LYS PRO VAL GLN ARG PHE MET LYS TYR GLN LEU LEU LEU LYS ASP PHE
        115                 120                 125
LEU LYS TYR TYR ASN ARG ALA GLY MET ASP THR ALA ASP LEU GLU GLN
    130                 135                 140
ALA VAL GLU VAL MET CYS PHE VAL PRO LYS ARG CYS ASN ASP MET MET
145                 150                 155                 160
THR LEU GLY ARG LEU ARG GLY PHE GLU GLY LYS LEU THR ALA GLN GLY
                165                 170                 175
LYS LEU LEU GLY GLN ASP THR PHE TRP VAL THR GLU PRO GLU ALA GLY
            180                 185                 190
GLY LEU LEU PHE SER ARG GLY ARG GLU ARG ARG VAL PHE LEU PHE GLU
        195                 200                 205
GLN ILE ILE ILE PHE SER GLU ALA LEU GLY GLY GLY VAL ARG GLY GLY
    210                 215                 220
THR LYS PRO GLY TYR VAL LEU GLN ASP GLN HIS
225                 230                 235
```

(2) INFORMATION FOR SEQ ID NO: 5:

(I) SEQUENCE CHARACTERISTICS:

(A) LENGTH: 2088 BASE PAIRS

(B) TYPE: NUCLEIC ACID

(C) STRANDEDNESS: SINGLE

(D) TOPOLOGY: LINEAR

(II) MOLECULE TYPE: CDNA

(XI) SEQUENCE DESCRIPTION: SEQ ID NO: 5:

ACGAGCCCGG CGCCGTCCCC GCCCCGCCCC CCGTGATTCC CCCTGCATGG CCGGCCCGGG 60
TGGGGGGCGC GGGGGGGCCC GGGCGCCATG CGGGGGGGGC ACAAAGGGGG TCGCTGTGCC 120
TGTCCCCGTG TGATCCGAAA AGTGCTGGCA AAATGCGGCT GCTGCTTCGC CCGGGGGGGA 180
CGTGAATCCT ATTCCATTGC GGGCAGTGAG GGGAGTATAT CGGCTTCTGC TGCCTCCGGT 240
CTGGCTGCCC CCTCTGGCCC CAGCTCTGGC CTCAGCTCTG GCCCCTGTTC CCCAGGCCCC 300
CCAGGGCCCG TCAGTGGCCT GAGGAGATGG TTGGATCATT CCAAACATTG TCTCAGTGTG 360
GAAACTGAGG CAGACAGTGG TCAGGCAGGA CCATATGAGA ACTGGATGTT GGAGCCAGCT 420
CTAGCCACAG GAGAGGAGCT GCCGGAACTG ACCTTGCTGA CCACACTGTT GGAGGGCCCT 480
GGAGATAAGA CGCAGCCACC TGAAGAGGAG ACTTTGTCCC AAGCCCCTGA GAGTGAGGAG 540
GAACAGAAGA AGAAGGCTCT GGAAAGGAGT ATGTATGTCC TGAGTGAACT GGTAGAAACA 600
GAGAAAATGT ACGTGGACGA CTTGGGGCAG ATTGTGGAGG GTTATATGGC CACCATGGCT 660
GCTCAGGGGG TCCCCGAGAG TCTTCGAGGC CGTGACAGGA TTGTGTTTGG GAATATCCAG 720
CAAATCTATG AGTGGCACCG AGACTATTTC TTGCAAGAGC TACAACGGTG TCTGAAAGAT 780
CCTGATTGGC TGGCTCAGCT ATTCATCAAA CACGAGCTCC GGCAGCAGCT GGGGCACCGC 840
CTGCAGCTGA ACGACCTCCT CATCAAACCT GTGCAGCGGA TCATGAAATA CCAGCTGCTG 900
CTCAAGGATT TTCTCAAGTA TTACAATAGA GCTGGGATGG ATACTGCAGA CCTAGAGCAA 960
GCTGTGGAGG TCATGTGCTT TGTGCCCAAG CGCTGCAACG ATATGATGAC GCTGGGGAGA 1020
TTGCGGGGAT TTGAGGGCAA ACTGACTGCT CAGGGGAAGC TCTTGGGCCA GGACACTTTC 1080
TGGGTCACCG AGCCTGAGGC TGGAGGGCTG CTGTCTTCCC GAGGTCGAGA GAGGCGCGTC 1140
TTCCTCTTTG AGCAAATCAT CATCTTCAGT GAAGCCCTGG GAGGAGGAGT GAGAGGTGGA 1200
ACACAGCCTG GATATGTATA CAAGAACAGC ATTAAGGTGA GCTGCCTGGG ACTGGAGGGG 1260
AACCTCCAAG GTGACCCTTG CCGCTTTGCA CTGACCTCCA GAGGGCCAGA GGGTGGGATC 1320
CAGCGCTATG TCCTGCAGGC TGCAGACCCT GCTATCAGTC AGGCCTGGAT CAAGCATGTG 1380
GCTCAGATCT TGGAGAGCCA ACGGGACTTC CTCAACGCAT TGCAGTCACC CATTGAGTAC 1440
CAGAGACGGG AGAGCCAGAC CAACAGCCTG GGGCGGCCAA GAGGGCCTGG AGTGGGGAGC 1500
CCTGGAAGAA TTCGGCTTGG AGATCAGGCC CAGGGCAGCA CACACACACC CATCAATGGC 1560
TCTCTCCCCT CTCTGCTGCT GTCACCCAAA GGGGAGGTGG CCAGAGCCCT CTTGCCACTG 1620
GATAAACAGG CCCTTGGTGA CATCCCCCAG GCTCCCCATG ACTCTCCTCC AGTCTCTCCA 1680
ACTCCAAAAA CCCCTCCCTG CCAAGCCAGA CTTGCCAAGC TGGATGAAGA TGAGCTGTAA 1740
CTGGTGAAAA CCATGGGGGT GGTGCTGACT CAGCCGCCTA TTCCCCAAGG AGCTTCAGGG 1800
CAGTCCTTCT GGCACTGCTC CAGAATTCCT CCTTCTTGGT GTGTCTGGAG GGTGGGCAAG 1860
GCTGGGAGGG ATATCAACTT GGAGGAGAAC ACCTAGACCC AAGGACTTTT TTCTGCCCAA 1920
GGAACACAGT TTCCTTCAGC TCCCATCCCT ATGCATGCAT CATGGTCCCC CCAAAAGGAG 1980
GATATGTGGG TGGGTGGGAG GGCTGGGGCA GGGGCCAGAT AGAAATTATT GGTTTTGTTT 2040
TTTAATTTTG TTTTTCCTGT TTTCTGAGAA TAAAGGTTTT GTTATATC 2088

(2) INFORMATION FOR SEQ ID NO: 6:

(I) SEQUENCE CHARACTERISTICS:

(A) LENGTH: 550 AMINO ACIDS

(B) TYPE: AMINO ACID

(C) STRANDEDNESS: SINGLE

(D) TOPOLOGY: LINEAR

(II) MOLECULE TYPE: PROTEIN

(XI) SEQUENCE DESCRIPTION: SEQ ID NO: 6:

```
MET ARG GLY GLY HIS LYS GLY GLY ARG CYS ALA CYS PRO ARG VAL ILE
 1               5                  10                  15
ARG LYS VAL LEU ALA LYS CYS GLY CYS CYS PHE ALA ARG GLY GLY ARG
            20                  25                  30
GLU SER TYR SER ILE ALA GLY SER GLU GLY SER ILE SER ALA SER ALA
        35                  40                  45
ALA SER GLY LEU ALA ALA PRO SER GLY PRO SER SER GLY LEU SER SER
    50                  55                  60
GLY PRO CYS SER PRO GLY PRO PRO GLY PRO VAL SER GLY LEU ARG ARG
65                  70                  75                  80
TRP LEU ASP HIS SER LYS HIS CYS LEU SER VAL GLU THR GLU ALA ASP
                85                  90                  95
SER GLY GLN ALA GLY PRO TYR GLU ASN TRP MET LEU GLU PRO ALA LEU
            100                 105                 110
ALA THR GLY GLU GLU LEU PRO GLU LEU THR LEU LEU THR THR LEU LEU
        115                 120                 125
GLU GLY PRO GLY ASP LYS THR GLN PRO PRO GLU GLU GLU THR LEU SER
    130                 135                 140
GLN ALA PRO GLU SER GLU GLU GLU GLN LYS LYS LYS ALA LEU GLU ARG
145                 150                 155                 160
SER MET TYR VAL LEU SER GLU LEU VAL GLU THR GLU LYS MET TYR VAL
                165                 170                 175
ASP ASP LEU GLY GLN ILE VAL GLU GLY TYR MET ALA THR MET ALA ALA
            180                 185                 190
GLN GLY VAL PRO GLU SER LEU ARG GLY ARG ASP ARG ILE VAL PHE GLY
        195                 200                 205
ASN ILE GLN GLN ILE TYR GLU TRP HIS ARG ASP TYR PHE LEU GLN GLU
    210                 215                 220
LEU GLN ARG CYS LEU LYS ASP PRO ASP TRP LEU ALA GLN LEU PHE ILE
```

225 230 235 240

LYS HIS GLU LEU ARG GLN GLN LEU GLY HIS ARG LEU GLN LEU ASN ASP
245 250 255

LEU LEU ILE LYS PRO VAL GLN ARG ILE MET LYS TYR GLN LEU LEU LEU
260 265 270

LYS ASP PHE LEU LYS TYR TYR ASN ARG ALA GLY MET ASP THR ALA ASP
275 280 285

LEU GLU GLN ALA VAL GLU VAL MET CYS PHE VAL PRO LYS ARG CYS ASN
290 295 300

ASP MET MET THR LEU GLY ARG LEU ARG GLY PHE GLU GLY LYS LEU THR
305 310 315 320

ALA GLN GLY LYS LEU LEU GLY GLN ASP THR PHE TRP VAL THR GLU PRO
325 330 335

GLU ALA GLY GLY LEU LEU SER SER ARG GLY ARG GLU ARG ARG VAL PHE
340 345 350

LEU PHE GLU GLN ILE ILE ILE PHE SER GLU ALA LEU GLY GLY GLY VAL
355 360 365

ARG GLY GLY THR GLN PRO GLY TYR VAL TYR LYS ASN SER ILE LYS VAL
370 375 380

SER CYS LEU GLY LEU GLU GLY ASN LEU GLN GLY ASP PRO CYS ARG PHE
385 390 395 400

ALA LEU THR SER ARG GLY PRO GLU GLY GLY ILE GLN ARG TYR VAL LEU
405 410 415

GLN ALA ALA ASP PRO ALA ILE SER GLN ALA TRP ILE LYS HIS VAL ALA
420 425 430

GLN ILE LEU GLU SER GLN ARG ASP PHE LEU ASN ALA LEU GLN SER PRO
435 440 445

ILE GLU TYR GLN ARG ARG GLU SER GLN THR ASN SER LEU GLY ARG PRO
450 455 460

ARG GLY PRO GLY VAL GLY SER PRO GLY ARG ILE ARG LEU GLY ASP GLN
465 470 475 480

ALA GLN GLY SER THR HIS THR PRO ILE ASN GLY SER LEU PRO SER LEU
485 490 495

LEU LEU SER PRO LYS GLY GLU VAL ALA ARG ALA LEU LEU PRO LEU ASP
500 505 510

LYS GLN ALA LEU GLY ASP ILE PRO GLN ALA PRO HIS ASP SER PRO PRO
515 520 525

VAL SER PRO THR PRO LYS THR PRO PRO CYS GLN ALA ARG LEU ALA LYS
530 535 540

LEU ASP GLU ASP GLU LEU
545 550